# EUROPÄISCHE PATENTANMELDUNG

(11) **EP 0 589 201 A1**
(43) Veröffentlichungstag der Anmeldung: **30.03.1994**
(21) Anmeldenummer: 93113069.4
(22) Anmeldetag: 16.08.1993
(51) Int. Cl.: C07D 239/22, C07D 251/20, A61K 31/505, A61K 31/53

(54) **Di- bzw. Triazindione**

(30) Priorität: 23.08.1992 DE 4227773
(71) Anmelder: MERCK PATENT GmbH, D-64293 Darmstadt (DE)
(72) Erfinder: Dorsch, Dieter, Dr., D-64372 Ober-Ramstadt (DE); Mederski, Werner, Dr., D-64390 Erzhausen (DE); Osswald, Mathias, Dr., D-64673 Zwingenberg (DE); Schelling, Pierre, Prof. Dr., D-64367 Mühltal (DE); Beier, Norbert, Dr., D-64354 Reinheim 5 (DE); Lues, Ingeborg, Dr., D-64297 Darmstadt (DE); Minck, Klaus-Otto, Dr., D-64372 Ober-Ramstadt (DE)

(57) **Zusammenfassung**

Neue Di- bzw. Triazindione der Formel I 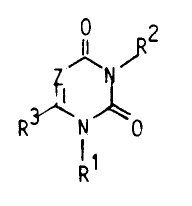
worin R¹, R², R³ und Z die in Patentanspruch 1 angegebene Bedeutung haben,
sowie deren Salze zeigen angiotensin II-antagonistische Eigenschaften und können zur Behandlung von Hypertension, Aldosteronismus, Herzinsuffizienz und erhöhtem Augeninnendruck sowie von Störungen des Zentralnervensystems verwendet werden.

## Beschreibung

Die Erfindung betrifft neue Di- bzw. Triazindione der Formel I 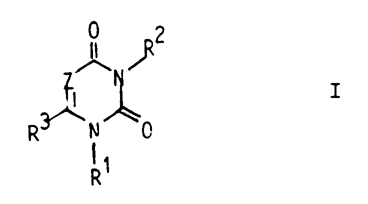
worin
- Z: CR⁴ oder N,
- R¹: 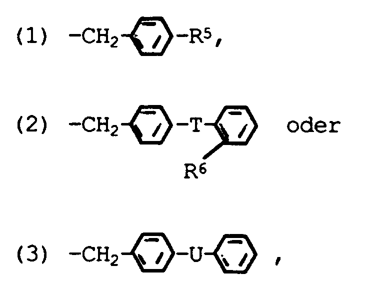
- R²: H, -C₁₋₁₀-Alkyl, -CₙH₂ₙ-COOR, -CₙH₂ₙ-CN, -CₙH₂ₙ-Ar, -CₙH₂ₙ-Het, -CₙH₂ₙ-CH=CH-Ar, -CₙH₂ₙ-CH=CH-Het, -CₙH₂ₙ-NR⁷R⁸, -C₂₋₆-Alkenyl, -CₙH₂ₙ-CO-N(R)₂, -CₙH₂ₙ-CO-R, -CₙH₂ₙ-CO-Ar, -CₘH₂ₘ-O-CON(R)₂ oder -CₘH₂ₘ-NR-CO-N(R)₂,
- R³: H, A, Alkenyl mit 2-6 C-Atomen, eine Alkylgruppe mit 1-6 C-Atomen, worin eine CH₂-Gruppe durch ein O- oder S-Atom ersetzt ist, oder C₃₋₇-Cycloalkyl,
- R⁴: sowie die Reste R unabhängig voneinander H, A oder C₃₋₇-Cycloalkyl,
- R⁵: COOR, CN oder 1H-5-Tetrazolyl,
- R⁶: COOR, CN, NO₂, NH₂, NHCOCF₃, NHSO₂CF₃ oder 1H-5-Tetrazolyl,
- R⁷: H, -CO-R oder -CO-CH(Ar)₂,
- R⁸: H,
- R⁷ und R⁸: zusammen auch -CO-(o-C₆H₄)-CO-,
- T: fehlt, -NR-CO-, -CO-NR- oder -CH=CH-,
- U: -CH=C(COOR)-, -CH=C(CN)-, -CH=C(1H-5-Tetrazolyl)-, -O-CH(COOR)- oder -NR-CH(COOR)-,
- m: 1, 2, 3, 4, 5, 6, 7, 8, 9 oder 10,
- n: 0, 1, 2, 3, 4, 5, 6, 7, 8, 9 oder 10,
- A: C₁₋₆-Alkyl,
- Ar: eine unsubstituierte oder eine ein- oder zweifach durch A, Hal, CF₃, OH, OA, COOH, COOA, CN, NO₂, NH₂, NHA und/oder N(A)₂ substituierte Phenyl- oder Naphthylgruppe,
- Het: einen fünf- oder sechsgliedrigen heteroaromatischen Rest mit 1 bis 4 N-, O- und/oder S-Atomen, der auch mit einem Benzol- oder Pyridinring kondensiert sein kann und
- Hal: F, Cl, Br oder I bedeuten,
sowie ihre Salze.

Ähnliche Verbindungen sind aus EP-A1-0 468 470 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der anciotensin II-abhängigen Hypertension, des Aldosteronismus, der Herzinsuffizienz und des erhöhten Augeninnendrucks sowie von Störungen des Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, von ischämischen peripheren Durchblutungsstörungen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, von gastrointestinalen Erkrankungen, Blasenerkrankungen, Lungenödemen, chronischer Bronchitis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression, Epilepsie, des Parkinson-Syndroms und/oder der Bulimie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Gegenstand der Erfindung sind die Verbindungen der Formel I, ihre Salze sowie ein Verfahren zur Herstellung dieser Verbindungen und ihrer Salze, dadurch gekennzeichnet, daß man
(a) eine Verbindung der Formel II

   E-R¹ II

   worin
   - E: Cl, Br, I oder eine freie oder reaktionsfähig funktionell abgewandelte OH-Gruppe bedeutet und
   - R¹: die oben angegebene Bedeutung hat,
   mit einer Verbindung der Formel III 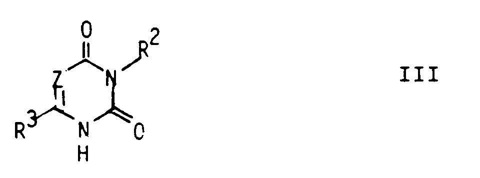 worin
   - Z, R², R³ und R⁴: die oben angegebene Bedeutung haben,
   umsetzt,
   oder
(b) zur Herstellung einer Verbindung der Formel I, worin T -NR-CO- oder -CO-NR- bedeutet, eine Verbindung der Formel IV 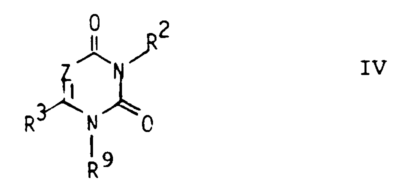 worin
   - R⁹: 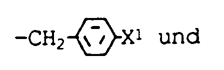
   - X¹: NH₂ oder COOH bedeuten, und
   - Z, R², R³ und R⁴: die oben angegebenen Bedeutungen haben,
   oder ein reaktionsfähiges Derivat dieser Verbindung mit einer Verbindung der Formel V 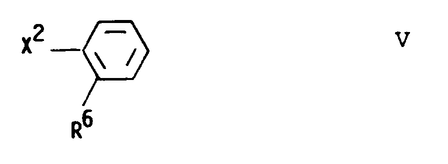 worin
   - X²: COOH (falls X¹ NH₂ ist) oder NH₂ (falls X¹ COOH ist) bedeutet und
   - R⁶: die oben angegebene Bedeutung hat,
   oder mit einem reaktionsfähigen Derivat dieser Verbindung umsetzt,
   oder
(c) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹ und/oder R² in einen oder mehrere andere Reste R¹ und/oder R² umwandelt und/oder eine Base oder Säure der Formel I in eines ihrer Salze umwandelt.

Vor- und nachstehend haben die Reste bzw. Parameter Z, R¹ bis R⁹, R, T, U, m, n, X¹, X², A, Ar, Het, Hal und E die bei den Formeln I bis V angegebenen Bedeutungen, falls nicht ausdrücklich etwas anderes angegeben ist.

In den vorstehenden Formeln bedeutet A insbesondere Alkyl mit 1-6, vorzugsweise 1, 2, 3, oder 4 C-Atomen, vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Dementsprechend ist der Rest OA vorzugsweise Methoxy, weiterhin Ethoxy, Propoxy, Isopropoxy, Butoxy, Isobutoxy, sek.-Butoxy oder tert.-Butoxy, der Rest SA bevorzugt Methylthio, weiterhin Ethylthio. Die Gruppe COOA ist bevorzugt Methoxycarbonyl oder Ethoxycarbonyl, ferner Propyloxycarbonyl, Isopropyloxycarbonyl, Butyloxycarbonyl, Isobutyloxycarbonyl. Die Gruppe NHA ist bevorzugt Methylamino oder Ethylamino. Die Gruppe N(A)₂ ist bevorzugt Dimethylamino oder Diethylamino.

Alkenyl ist vorzugsweise Vinyl, Allyl oder 1-Propen-1-yl.

Eine Alkylgruppe, worin eine CH₂-Gruppe durch ein O- oder S-Atom ersetzt ist, ist vorzugsweise OA, z.B. Methoxy, oder SA, z.B. Methylthio, ferner auch z.B. Methoxymethyl, Ethoxymethyl, 2-Methoxyethyl, 2-Ethoxyethyl, Methylthiomethyl, Ethylthiomethyl, 2-Methylthioethyl, 2-Ethylthioethyl.

Cycloalkyl ist vorzugsweise Cyclopropyl, ferner Cyclobutyl, Cyclopentyl, Cyclohexyl, Cycloheptyl, aber auch z.B. 4-Methyl-cyclohexyl.

Hal bedeutet vorzugsweise F, Cl oder Br, aber auch J.

Der Rest Ar ist vorzugsweise eine unsubstituierte Phenylgruppe, weiterhin bevorzugt eine in p-Stellung monosubstituierte, aber auch in o- oder m-Stellung monosubstituierte Phenylgruppe. Bevorzugte Substituenten sind OA, COOH, COOA und NO₂. Dementsprechend ist Ar bevorzugt Phenyl, o-, m- oder (insbesondere) p-Methoxyphenyl, o-, m- oder (insbesondere) p-Carboxyphenyl, o-, m- oder (insbesondere) p-Methoxycarbonylphenyl, o-, m- oder (insbesondere) p-Ethoxycarbonylphenyl, o-, m- oder (insbesondere) p-Nitrophenyl, ferner bevorzugt o-, m- oder (insbesondere) p-Aminophenyl, o-, m- oder (insbesondere) p-Dimethylaminophenyl, o-, m- oder (insbesondere) p-Diethylaminophenyl, o-, m- oder p-Tolyl, o-, m- oder p-Trifluormethylphenyl, o-, m- oder p-Hydroxyphenyl, o-, m- oder p-Fluorphenyl, o-, m- oder p-Chlorphenyl, o-, m- oder p-Bromphenyl, o-, m- oder p-Iodphenyl, o-, m- oder p-Cyanphenyl, o-, m- oder p-Methylaminophenyl, 1- oder 2-Naphthyl.

Het enthält vorzugsweise ein bis vier N-Atome und/oder ein O-Atom und/oder ein S-Atom und ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

In den Begriff "Het" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z. B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl, 4-Methyl-5-pyrazolyl, 4- oder 5-Methyl-3-isoxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Der Rest Z ist vorzugsweise CH, ferner bevorzugt CA, z.B. CCH₃ oder N.

Der Rest T fehlt vorzugsweise; weiterhin ist T bevorzugt -NH-CO-, -N(CH₃)-CO-, -CO-NH-, -CO-N(CH₃)- oder -CH=CH-.

Der Rest U ist vorzugsweise -CH=C(CN)- oder -CH=C(1H-5-Tetrazolyl)-, ferner bevorzugt -CH=C(COOH)-, -CH=C(COOCH₃)-, -CH=C(COOC₂H₅)-, -O-CH(COOH)-, -O-CH(COOCH₃)-, -O-CH(COOC₂H₅)-, -NH-CH(COOH)-, -N(CH₃)-CH(COOH)-, -NH-CH(COOCH₃)-, -N(CH₃)-CH(COOCH₃)-, -NH-CH(COOC₂H₅)- oder -N(CH₃)-CH(COOC₂H₅)-.

Die Reste R stehen unabhängig voneinander bevorzugt für H, CH₃ oder C₂H₅.

Der Rest R¹ ist vorzugsweise 2'-Cyan-biphenylyl-4-methyl, 2'-Carboxy-biphenylyl-4-methyl, 2'-(1H-5-Tetrazolyl)-biphenylyl-4-methyl, p-(2-Cyan-2-phenyl-vinyl)-benzyl, p-[2-Phenyl-2-(1H-5-tetrazolyl)-vinyl]-benzyl oder p-Carboxybenzyl.

Der Rest R² ist vorzugsweise H; A, vor allem Methyl, Ethyl, Propyl, Isopropyl, Butyl oder Isobutyl; -(CH₂)ₙ-COOR, insbesondere -CH₂-COOR, vor allem Carboxymethyl, Methoxycarbonylmethyl oder Ethoxycarbonylmethyl, ferner bevorzugt -COOR wie Carboxy, Methoxycarbonyl, Ethoxycarbonyl, oder -CH₂CH₂-COOR, vor allem 2-Carboxyethyl, 2-Methoxycarbonyl-ethyl, 2-Ethoxycarbonyl-ethyl; - (CH₂)ₙ-CN, insbesondere Cyan, Cyanmethyl, 2-Cyanethyl, (CH₂)ₙ-Ar, insbesondere CH₂-Ar wie Benzyl, o-, m- oder p-Carboxybenzyl, o-, m- oder p-Methoxycarbonylbenzyl, o-, m- oder p-Ethoxycarbonylbenzyl, ferner bevorzugt -Ar wie Phenyl, o-, m- oder p-Carboxyphenyl, o-, m- oder p-Methoxycarbonylphenyl, o-, m- oder p-Ethoxycarbonylphenyl oder -CH₂CH₂-Ar wie 2-Phenylethyl, 2-(o-, 2-(m- oder 2-(p-Carboxyphenyl)-ethyl; -(CH₂)ₙ-Het, insbesondere -CH₂-Het wie 2- oder 3-Thienylmethyl, 2-, 3- oder 4-Pyridylmethyl, 1H-5-Tetrazolyl-methyl, ferner bevorzugt -Het wie 2- oder 3-Thienyl, 2-, 3- oder 4-Pyridyl, 1H-5-Tetrazolyl, oder -CH₂CH₂-Het wie 2-(2- oder 2-(3-Thienyl)-ethyl, 2-(2-, 2-(3- oder 2-(4-Pyridyl)-ethyl, 2-(1H-5-Tetrazolyl)-ethyl; -(CH₂)ₙ-CH=CH-Ar, insbesondere -CH₂-CH=CH-Ar wie Cinnamyl, ferner bevorzugt -CH=CH-Ar wie 2-Phenylvinyl, oder -CH₂CH₂-CH=CH-Ar wie 4-Phenyl-3-buten-1-yl; -(CH₂)ₙ-CH=CH-Het, insbesondere -CH₂-CH=CH-Het wie 3-(1H-5-Tetrazolyl)-2-propen-1-yl; -(CH₂)ₙ-CO-N(R)₂, insbesondere -CH₂-CO-N(R)₂ wie Carbamoylmethyl, N-Methyl-carbamoylmethyl, N-Ethyl-carbamoylmethyl, N,N-Dimethylcarbamoylmethyl, N,N-Diethyl-carbamoylmethyl, ferner bevorzugt -CO-N(R)₂ wie Carbamoyl, N-Methyl-carbamoyl, N,N-Dimethyl-carbamoyl, oder -CH₂CH₂-CO-N(R)₂ wie 2-Carbamoyl-ethyl, 2-N,N-Dimethylcarbamoyl-ethyl; -(CH₂)ₙ-CO-R, insbesondere -CH₂-CO-R wie Formylmethyl, 2-Oxopropyl, ferner bevorzugt -CO-R wie Formyl, Acetyl, Propionyl oder Butyryl, oder -CH₂CH₂-CO-R wie 2-Formylethyl, 3-Oxobutyl; -(CH₂)ₙ-CO-Ar, insbesondere -CH₂-CO-Ar wie Phenacyl, ferner bevorzugt -CO-Ar wie Benzoyl, oder -CH₂CH₂-CO-Ar wie 3-Oxo-3-phenylpropyl; -(CH₂)ₘ-O-CO-N(R)₂, insbesondere -CH₂-O-CO-N(R)₂ wie Carbamoyloxymethyl, N-Methylcarbamoyloxymethyl, N,N-Dimethyl-carbamoyloxymethyl, oder -CH₂CH₂-O-CO-N(R)₂ wie 2-Carbamoyl- oxy-ethyl, 2-(N-Methylcarbamoyloxy)-ethyl, 2-(N,N-Dimethyl-carbamoyloxy)-ethyl; -(CH₂)ₘ-NR-CO-N(R)₂, insbesondere -CH₂-NR-CO-N(R)₂ wie Ureidomethyl, N'-Methylureidomethyl, N-Methylureidomethyl, N,N'-Dimethylureidomethyl, N',N'-Dimethylureidomethyl, N,N',N'-Trimethylureidomethyl, oder -CH₂CH₂-NR-CO-N(R)₂ wie 2-Ureidoethyl.

Der Rest R³ ist vorzugsweise A, vor allem Ethyl, Propyl oder Butyl, oder Cycloalkyl, vor allem Cyclopropyl.

Der Rest R⁴ ist vorzugsweise H, ferner bevorzugt A, insbesondere Methyl oder Ethyl.

Der Rest R⁵ ist vorzugsweise COOH, ferner bevorzugt COOCH₃, COOC₂H₅, CN oder 1H-5-Tetrazolyl.

R⁷ ist vorzugsweise H, Acetyl oder Diphenylacetyl.

R⁸ ist vorzugsweise H.

Die Gruppe NR⁷R⁸ ist ferner bevorzugt Phthalimido.

Der Rest R⁶ ist vorzugsweise COOH, COOCH₃, COOC₂H₅, CN oder 1H-5-Tetrazolyl.

Der Parameter m ist vorzugsweise 1 oder 2, der Parameter n ist vorzugsweise 0, 1 oder 2. Die Gruppe CₘH₂ₘ steht dabei bevorzugt für -(CH₂)ₘ-, insbesondere für -CH₂- oder -CH₂CH₂-, die Gruppe CₙH₂ₙ bevorzugt für -(CH₂)ₙ-, insbesondere für -CH₂- oder -CH₂CH₂-.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Die Verbindungen der Formel I umschließen die bevorzugten Uracile der Formel Ia (= I, Z = CR⁴; 1-R³-3-R²-5-R⁴-6-R³-1,2,3,4-tetrahydro-1,3-diazin-2,4-dione) sowie die Triazindione der Formel Ib (= I, Z = N; 1-R¹-3-R²-6-R³-1,2,3,4-tetrahydro-1,3,5-triazin-2,4-dione).

Gegenstand der Erfindung sind insbesondere diejenigen Verbindungen der Formeln I, Ia und Ib, in denen mindestens einer der genannten Reste eine der vorstehend angegebenen bevorzugten Bedeutungen hat. Einige bevorzugte Gruppen von Verbindungen können durch die folgenden Teilformeln Ic bis Ibe ausgedrückt werden, die den Formeln I, Ia oder Ib entsprechen und worin die nicht näher bezeichneten Reste die bei Formel I angegebenen Bedeutungen haben, worin jedoch
- in Ic, Iac und Ibc R¹: 2'-Cyan-biphenylyl-4-methyl, 2'-(1H-5-Tetrazolyl)-biphenylyl-4-methyl, p-Carboxybenzyl oder p-[2-Phenyl-2-(1H-5-tetrazolyl)-vinyl]-benzyl bedeutet;
- in Id, Iad und Ibd R²: A, CH₂COOR, CH₂CN, CH₂C₆H₅, o-COOR-benzyl, CH₂(1H-5-tetrazolyl) oder CH₂CON(R)₂ bedeutet;
- in Ie, Iae und Ibe R¹: 2'-Cyan-biphenylyl-4-methyl; 2'-(1H-5-Tetrazolyl)-biphenylyl-4-methyl, p-Carboxybenzyl oder p-[2-Phenyl-2-(1H-5-tetrazolyl)-vinyl]-benzyl und
- R²: A, CH₂COOR, CH₂CN, CH₂C₆H₅, o-COOR-benzyl, CH₂-(1H-5-tetrazolyl) oder CH₂CON(R)₂ bedeuten.

Insbesondere sind bevorzugt Verbindungen aller vorstehend genannten Formeln, in denen zusätzlich R³ A oder C₃₋₇-Cycloalkyl bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A1-0 468 470 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃ONa oder K-tert.-Butylat in einem Alkohol wie CH₃OH, in einem Ether wie Tetrahydrofuran (THF) oder in einem Amid wie Dimethylformamid (DMF) oder mit einem Alkalimetallhydrid wie NaH oder einem Alkalimetallalkoholat in DMF, und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallhydroxide wie NaOH oder KOH, Alkalimetallcarbonate wie Na₂CO₃ oder K₂CO₃ oder Alkalimetallhydrogencarbonate wie NaHCO₃ oder KHCO₃. Man kann auch zweiphasig arbeiten, z.B. in Dichlormethan/Wasser, wobei man zweckmäßig einen Phasentransfer-Katalysator zusetzt, z.B. Tetrabutylammoniumbromid.

Verwendet man eine Verbindung der Formel III, worin R² = H ist, so erhält man bei der Reaktion mit II in der Regel Gemische, die jedoch leicht trennbar sind, z.B. chromatographisch. Das Mengenverhältnis und die Art der Produkte können durch Abwandlung der Reaktionsbedingungen gesteuert werden.

Säureamide der Formel I (T = -NR-CO- oder -CO-NR-) sind ferner erhältlich durch Umsetzung von Verbindungen der Formel IV (oder ihrer reaktionsfähigen Derivate) mit Verbindungen der Formel V (oder ihrer reaktionsfähigen Derivate).

Als reaktionsfähige Derivate der Carbonsäuren der Formel IV und V (X¹ bzw. X² = COOH) eignen sich vorteilhaft die entsprechenden Chloride, Bromide oder Anhydride. Die Umsetzung erfolgt zweckmäßig in Gegenwart eines inerten Lösungsmittels, z.B. eines halogenierten Kohlenwasserstoffs wie Dichlormethan, Chloroform, Trichlorethen oder 1,2-Dichlorethan oder eines Ethers wie THF oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Weiterhin kann man eine Verbindung der Formel I durch Solvolyse (z.B. Hydrolyse) oder Hydrogenolyse aus einem ihrer funktionellen Derivate in Freiheit setzen.

So kann man Carbonsäuren der Formel I, worin U -O-CH(COOH), -NH-CH(COOH), -NA-CH(COOH) oder -CH=C(COOH) oder R⁵ oder R⁶ COOH und/oder R² -CₙH₂ₙ-COOH und/oder Ar eine ein- oder zweifach durch COOH substituierte Phenyl- oder Naphthylgruppe bedeutet, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.

Ferner ist es möglich, nach einer der angegebenen Methoden eine Verbindung herzustellen, die der Formel I entspricht, aber an Stelle einer 5-Tetrazolylgruppe eine in 1-Stellung (oder 2-Stellung) funktionell abgewandelte (durch eine Schutzgruppe geschützt) 1H- (oder 2H)-5-Tetrazolylgruppe enthält, und diese Schutzgruppe zum Schluß abzuspalten. Als Schutzgruppen eignen sich beispielsweise: Triphenylmethyl, abspaltbar mit HCl oder Ameisensäure in einem inerten Lösungsmittel oder Lösungsmittelgemisch, z.B. Ether/Dichlormethan/Methanol; 2-Cyanethyl, abspaltbar mit NaOH in Wasser/THF; p-Nitrobenzyl, abspaltbar mit H₂/Raney-Nickel in Ethanol (vgl. EP-A2-0 291 969).

Die Ausgangsstoffe, insbesondere diejenigen der Formeln II und V, sind teilweise bekannt. Falls sie nicht bekannt sind, können sie nach bekannten Methoden in Analogie zu bekannten Stoffen hergestellt werden.

Verbindungen der Formel III (Z = CR⁴) sind z.B. erhältlich durch Reaktion von Ketoestern der Formel R³-CO-CH₂-COOA mit Harnstoffen der Formel R²-NHCONH₂ (vgl. US 3 235 357), oder durch Reaktion dieser Ketoester mit Thioharnstoff zu 6-R³-2-thiouracilen (= 4-Oxo-5-R⁴-6-R³-2-thioxo-1,2,3,4-tetrahydropyrimidinen), anschließende Reaktion mit Verbindungen der Formel R²-E oder Dialkylsulfaten zu 4-Oxo-2-R²S-3-R²-5-R⁴-6-R³-3,4-dihydro-pyrimidinen und nachfolgende Hydrolyse, z.B. mit Salzsäure.

Verbindungen der Formel IV sind z.B. erhältlich durch Umsetzung von Verbindungen der Formel III mit Verbindungen, die der Formel R⁹-E entsprechen, worin aber der Rest X¹ in geschützter Form vorliegt, die Aminogruppe z.B. durch eine Aminoschutzgruppe (z.B. Benzyl, A-O-CO- oder Benzyloxycarbonyl) oder die Carboxylgruppe eine Carboxyl-Schutzgruppe (z.B. in Form einer Estergruppe) geschützt ist, sowie nachfolgende Abspaltung der Schutzgruppe.

Es ist ferner möglich, eine Verbindung der Formel I in eine andere Verbindung der Formel I umzuwandeln, indem man einen oder mehrere der Reste R¹ und/oder R² in andere Reste R¹ und/oder R² umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Kupfer(I)cyanid) durch CN-Gruppen ersetzt und/oder Nitrilgruppen zu COOH-Gruppen oder zu CONH₂-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder freie Hydroxy- und/oder NH-Gruppen mit einem unsubstituierten oder substituierten Alkylhalogenid oder mit Aldehyden wie Formaldehyd in Gegenwart eines Reduktionsmittels wie NaBH₄ oder Ameisensäure alkylieren, zweckmäßig in einem inerten Lösungsmittel wie Dichlormethan oder THF und/ oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine COOA-Gruppe enthält, in die entsprechende Verbindung der Formel I umgewandelt werden, die stattdessen eine COOH-Gruppe enthält. Estergruppen können z.B. mit NaOH oder KOH in Methanol, Wasser, Wasser-THF oder Wasser-Dioxan bei Temperaturen zwischen 0 und 100° verseift werden.

Umsetzung von Nitrilen der Formel I (R² oder R³ = -CₙH₂ₙ-CN; oder R⁴ oder R⁵ = CN) mit Derivaten der Stickstoffwasserstoffsäure führt zu Tetrazolen der Formel I (R² oder R³ = -Cₙ-H₂ₙ-1H-5-Tetrazolyl; R⁴ oder R⁵ = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°. Anschließend wird die Trialkylzinn-Gruppe abgespalten, entweder durch Behandeln mit Salzsäure, z.B. in Dioxan, oder mit Alkali, z.B. in Ethanol/Wasser, oder mit Ameisensäure, z.B. in Methanol, oder durch Chromatographie an einer Kieselgel-Säule, z.B. mit Ethylacetat/Methanol.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel, z.B. Ethanol, und anschließendes Eindampfen. Für diese Umsetzung kommen insbesondere Säuren in Frage, die physiologisch unbedenkliche Salze liefern. So können anorganische Säuren verwendet werden, z.B. Schwefelsaure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgas oder Treibgasgemisch (z.B. Kohlenwasserstoffen wie Propan oder Butan oder Fluorkohlenwasserstoffen wie Heptafluorpropan) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 100 mg/kg, insbesondere 1 und 50 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. FAB = Massenspektrum, nach der "fast atom bombardment"-Methode.

### Beispiel 1

Zu einer Lösung von 16,8 g 3-Methyl-6-propyl-uracil ["IIIa"; erhältlich z.B. durch Reaktion von Ethyl-butyrylacetat mit Thioharnstoff in Ethanol in Gegenwart von Na-ethanolat zu 6-Propyl-2-thiouracil (= 4-Oxo-6-propyl-2-thioxo-1,2,3,4-tetrahydropyrimidin; F. 279°), Methylierung mit Dimethylsulfat/NaOH/Wasser zu 3-Methyl-2-methylthio-4-oxo-6-propyl-3,4-dihydropyrimidin und Behandeln mit siedender konzentrierter Salzsäure] in 400 ml Dichlormethan werden 13,3 g Tetrabutylammoniumbromid, dann eine Lösung von 8 g NaOH in 120 ml Wasser, dann 27,2 g 4'-Brommethyl-2-cyanbiphenyl ("IIa") gegeben. Man rührt das Gemisch 32 Std. bei 40°, arbeitet wie üblich auf (Kieselgel; Petrolether/Ethylacetat 6:4) und erhält 1-(2'-Cyan-biphenylyl-4-methyl)-3-methyl-6-propyl-uracil, F. 147°.

Analog erhält man:
aus IIIa und p-Brommethyl-benzoesäuremethylester:
1-(p-Methoxycarbonylbenzyl)-3-methyl-6-propyl-uracil;
aus IIIa und p-(2-Cyan-2-phenyl-vinyl)-benzylbromid:
1-[p-(2-Cyan-2-phenyl-vinyl)-benzyl]-3-methyl-6-propyl-uracil;
aus IIIa und 4'-Brommethyl-2-nitro-biphenyl:
1-(2'-Nitro-biphenylyl-4-methyl)-3-methyl-6-propyl-uracil, FAB 380;
aus 6-Butyl-3-methyl-uracil und IIa:
6-Butyl-1-(2'-cyan-biphenylyl-4-methyl)-3-methyl-uracil, FAB 374;
aus 6-Cyclopropyl-3-methyl-uracil und IIa:
1-(2'-Cyan-biphenylyl-4-methyl)-6-cyclopropyl-3-methyl-uracil, FAB 358;
aus 6-Cyclohexyl-3-methyl-uracil und IIa:
1-(2'-Cyan-biphenylyl-4-methyl)-6-cyclohexyl-3-methyl-uracil, FAB 400;
aus 3,5-Dimethyl-6-propyl-uracil und IIa:
1-(2'-Cyan-biphenylyl-4-methyl)-3,5-dimethyl-6-propyl-uracil, FAB 374;
aus 3-Butyl-6-propyl-uracil und IIa:
3-Butyl-1-(2'-cyan-biphenylyl-4-methyl)-6-propyl-uracil, FAB 402;
aus 3-Phenyl-6-propyl-uracil und IIa:
1-(2'-Cyan-biphenylyl-4-methyl)-3-phenyl-6-propyl-uracil, FAB 394;
aus 3-Benzyl-6-propyl-uracil (F. 154°; aus Ethyl-butyrylacetat und N-Benzylharnstoff) und IIa:
3-Benzyl-1-(2'-cyan-biphenylyl-4-methyl)-6-propyl-uracil, F. 196°;
aus 3-Methoxycarbonylmethyl-6-propyl-uracil und IIa:
1-(2'-Cyan-biphenylyl-4-methyl)-3-methoxycarbonylmethyl-6-propyl-uracil, FAB 418;
aus 3-o-Methoxycarbonyl-benzyl-6-propyl-uracil und IIa:
1-(2'-Cyan-biphenylyl-4-methyl)-3-o-methoxycarbonyl-benzyl-6-propyl-uracil, FAB 494;
aus 3-N,N-Dimethylcarbamoylmethyl-6-propyl-uracil und IIa:
1-(2'-Cyan-biphenylyl-4-methyl)-3-N,N-dimethylcarbamoylmethyl-6-propyl-uracil, FAB 431;
aus 3-Allyl-6-propyl-uracil und IIa:
3-Allyl-1-(2'-cyan-biphenylyl-4-methyl)-6-propyl-uracil, FAB 386;
aus 3-Cyanmethyl-6-propyl-uracil und IIa:
1-(2'-Cyan-biphenylyl-4-methyl)-3-cyanmethyl-6-propyl-uracil, FAB 385.

### Beispiel 2

Ein Gemisch von 0,01 Mol 6-Butyl-3-methyl-2,4-dioxo-1,2,3,4-tetrahydro-1,3,5-triazin (erhältlich durch Reaktion von Valeriansäureanhydrid mit Dicyandiamid zu 6-Butyl-4-imino-2-oxo-1,2,3,4-tetrahydro-1,3,5-triazin analog R. Andreasch, Monatshefte für Chemie, 43, 145 ff. (1927), Hydrolyse mit H₂SO₄ zu 6-Butyl-2,4-dioxo-1,2,3,4-tetrahydro-1,3,5-triazin (F. 185°) und Umsetzung mit O-Methyl-N,N'-dicyclohexylisoharnstoff in DMF bei 100°), 0,01 Mol IIa, 0,011 Mol Kalium-tert.-butanolat und 400 ml THF wird 18 Std. bei 20° gerührt. Man dampft ein, arbeitet wie üblich auf (Kieselgel; Dichlormethan/Methanol 95:5) und erhält 6-Butyl-1-(2'-cyan-biphenylyl-4-methyl)-3-methyl-2,4-dioxo-1,2,3,4-tetrahydro-1,3,5-triazin, F. 57°.

### Beispiel 3

Ein Gemisch von 1 g 1-p-Aminobenzyl-3-methyl-6-propyl-uracil (erhältlich durch Reaktion von IIIa mit p-BOC-aminobenzylbromid zu 1-p-BOC-aminobenzyl-3-methyl-6-propyl-uracil und anschließende Abspaltung der Schutzgruppe), 0,6 g Phthalsäureanhydrid und 40 ml CHCl₃ wird 16 Std. bei 20° gerührt. Das ausgefallene Phthalsäuremono-p-(3-methyl-6-propyl-uracilyl-1-methyl)-anilid wird abfiltriert.

### Beispiel 4

Ein Gemisch von 1,73 g 1-p-Aminobenzyl-3-methyl-6-propyl-uracil, 3 ml Triethylamin, 0,5 g 4-Dimethylaminopyridin und 120 ml CH₂Cl₂ wird auf 5° gekühlt und tropfenweise mit einer Lösung von 2,88 g o-Trifluormethansulfonamido-benzoylchlorid in 20 ml CH₂Cl₂ versetzt. Man rührt noch 16 Std. bei 20°, dampft ein, arbeitet wie üblich auf und erhält 3-Methyl-6-propyl-1-[4-(o-trifluormethansulfonamidobenzamido)-benzyl]-uracil.

### Beispiel 5

Ein Gemisch von 3,02 g 1-p-Carboxybenzyl-3-methyl-6-propyl-uracil, 12 g SOCl₂ und 35 ml CHCl₃ wird 6 Std. gekocht und eingedampft. Das erhaltene rohe Säurechlorid wird durch mehrfaches Lösen in Toluol und Eindampfen von Resten SOCl₂ befreit und in 80 ml THF gelöst. Man tropft diese Lösung zu einer Lösung von 1,37 g Anthranilsäure und 0,8 g NaOH in 100 ml Wasser, rührt 24 Std. und säuert mit HCl bis pH 5 an. Nach üblicher Aufarbeitung erhält man 1-[4-(o-Carboxyanilinocarbonyl)-benzyl]-3-methyl-6-propyl-uracil.

### Beispiel 6

a) Eine Lösung von 16,8 g 3-Methyl-6-propyl-uracil (IIIa) in 300 ml DMF wird unter Rühren mit 11 g Kalium-tert.butylat, dann nach 30 Minuten mit 54,5 g 4'-Brommethyl-2-[1(oder 2)-triphenylmethyl-1H(oder 2H)-5-tetrazolyl]-biphenyl (vgl. EP-A2-0 392 317; dort als "-1-triphenylmethyl-1H-" bezeichnet, Struktur jedoch nicht bewiesen) versetzt und 3 Std. bei 20° gerührt. Nach üblicher Aufarbeitung erhält man 3-Methyl-1-[2'-(1-triphenylmethyl-1H-5-tetrazolyl)-biphenylyl-4-methyl]-6-propyl-uracil.
b) Eine Lösung von 6,33 g des nach a) erhaltenen Produkts in 30 ml Dichlormethan und 30 ml Methanol wird mit 20 ml etherischer Salzsäure versetzt und 3 Std. bei 20° gerührt. Man dampft ein, arbeitet wie üblich auf und erhält nach chromatographischer Abtrennung des gebildeten Triphenylcarbinols 3-Methyl-6-propyl-1-[2'-(1H-5-tetrazolyl)-biphenyl-4-methyl]-uracil, Dihydrat, F. 119°

### Beispiel 7

Eine Lösung von 1 g 1-p-Benzyloxycarbonylbenzyl-3-methyl-6-propyl-uracil (erhältlich aus IIIa und p-Brommethyl-benzoesäure-benzylester) in 25 ml Ethanol wird an 0,5 g 5%ig. Pd-Kohle bei 20° und 1 bar bis zur Aufnahme der berechneten Menge H₂ hydriert. Man filtriert ab, dampft ein und erhält nach chromatographischer Reinigung 1-p-Carboxybenzyl-3-methyl-6-propyl-uracil. Als Nebenprodukt entsteht IIIa.

### Beispiel 8

Eine Lösung von 1 g 3-Methoxycarbonylmethyl-6-propyl-1-[2'-(1H-5-tetrazolyl)-biphenylyl-4-methyl]-uracil (s. Beispiel 10) in 9 ml 1 N wässeriger Natronlauge und 60 ml Dioxan wird 48 Std. bei 20° gerührt. Man konzentriert die Lösung, nimmt den Rückstand in Wasser auf, wäscht mit CH₂Cl₂, säuert mit 1 N Salzsäure an und arbeitet wie üblich auf. Man erhält 3-Carboxymethyl-6-propyl-1-[2'-(1H-5-tetrazolyl)-biphenylyl-4-methyl]-uracil.

Analog erhält man durch Verseifung der entsprechenden Methylester:
3-o-Carboxybenzyl-6-propyl-1-[2'-(1H-5-tetrazolyl)-biphenylyl-4-methyl]-uracil
1-p-Carboxybenzyl-3-methyl-6-propyl-uracil, Monohydrat, F. 141°.

### Beispiel 9

a) Eine Lösung von 1 g 1-(2'-Nitro-biphenylyl-4-methyl)-3-methyl-6-propyl-uracil in 30 ml Ethanol wird an 1 g Raney-Nickel bis zum Stillstand der H₂-Aufnahme bei 20° hydriert. Man filtriert, dampft ein und erhält 1-(2'-Amino-biphenylyl-4-methyl)-3-methyl-6-propyluracil.
b) Eine Lösung von 2,82 g Trifluormethansulfonsäureanhydrid in 10 ml CH₂Cl₂ wird zugetropft zu einer Lösung von 3,49 g 1-(2'-Aminobiphenylyl-4-methyl)-3-methyl-6-propyl-uracil und 1,01 g Triethylamin in 30 ml CH₂Cl₂ bei -50 bis -60°. Man läßt das Gemisch sich auf 20° erwärmen, gießt in verdünnte Essigsäure und erhält nach üblicher Aufarbeitung 3-Methyl-6-propyl-1-(2'-trifluormethansulfonamidobiphenylyl-4-methyl)-uracil.

### Beispiel 10

Eine Suspension von 3,59 g 1-(2'-Cyan-biphenylyl-4-methyl)-3-methyl-6-propyl-uracil und 6,2 g Trimethylzinnazid in 140 ml Toluol wird 9 Tage gekocht. Man dampft ein, löst den Rückstand in 1 N NaOH-Lösung, versetzt mit konzentrierter Salzsäure und filtriert das erhaltene 3-Methyl-6-propyl-1-[2'-(1H-5-tetrazolyl)-biphenylyl-4-methyl]-uracil-dihydrat ab; F. 119°.

Analog erhält man aus den entsprechenden Nitrilen:
3-Methyl-6-propyl-1-[p-(2-phenyl-2-(1H-5-tetrazolyl)-vinyl)-benzyl]-uracil
6-Butyl-3-methyl-1-[2'-(1H-5-tetrazolyl)-biphenylyl-4-methyl]-uracil, Nonahydrat, F.74°
6-Cyclopropyl-3-methyl-1-[2'-(1H-5-tetrazolyl)-biphenylyl-4-methyl]-uracil, Trihydrat, FAB 401
6-Cyclohexyl-3-methy-1-[2'-(1H-5-tetrazolyl)-biphenylyl-4-methyl]-uracil
3,5-Dimethyl-6-propyl-1-[2'-(1H-5-tetrazolyl)-biphenylyl-4-methyl]-uracil, FAB 417
3-Butyl-6-propyl-1-[2'-(1H-5-tetrazolyl)-biphenylyl-4-methyl]-uracil, Tetrahydrat, F. 147°
3-Phenyl-6-propyl-1-[2'-(1H-5-tetrazolyl)-biphenylyl-4-methyl]-uracil
3-Benzyl-6-propyl-1-[2'-(1H-5-tetrazolyl)-biphenylyl-4-methyl]-uracil, Dihydrat, F. 107°
3-Methoxycarbonylmethyl-6-propyl-1-[2'-(1H-5-tetrazolyl)-biphenylyl-4-methyl]-uracil
3-o-Methoxycarbonyl-benzyl-6-propyl-1-[2'-(1H-5-tetrazolyl)-biphenylyl-4-methyl]-uracil, Monohydrat, F. 146°; K-Salz, Dekahydrat, F. 227°
3-N,N-Dimethylcarbamoylmethyl-6-propyl-1-[2'-(1H-5-tetrazolyl)-biphenylyl-4-methyl]-uracil, Trihydrat, FAB 474; K-Salz, Nonahydrat, F. 183°
3-Allyl-6-propyl-1-[2'-(1H-5-tetrazolyl)-biphenylyl-4-methyl]-uracil, Monohydrat, F. 84°
6-Butyl-3-methyl-2,4-dioxo-1-[2'-(1H-5-tetrazolyl)-biphenylyl-4-methyl]-1,2,3,4-tetrahydro-1,3,5-triazin, Oktahydrat, F. 73°.

### Beispiel 11

Analog Beispiel 1 erhält man aus den entsprechenden in 1-Stellung unsubstituierten Uracilen mit IIa die nachstehenden 1-(2'-Cyanbiphenylyl-4-methyl)-uracile:
3-Methyl-6-ethyl-, FAB 346
3-o-Methoxycarbonyl-benzyl-6-butyl-, FAB 508
3-(2-Phthalimido-ethyl)-6-propyl, FAB 519.

### Beispiel 12

a) Zu einer Lösung von 7,9 g 3-(2-Phthalimido-ethyl)-6-propyl-uracil (erhältlich durch Umsetzung von 1,6-Dihydro-2-methoxy-4-propyl-pyrimidin-6-on mit 2-Phthalimidoethyl-bromid in Gegenwart von Cäsiumcarbonat zu 1,6-Dihydro-2-methoxy-3-(2-phthalimido-ethyl)-4-propyl-pyrimidin-6-on und Hydrolyse mit 1 N HCl) in 100 ml DMF gibt man 7,9 g Caesiumcarboant, rührt 1 Std. bei 20°, gibt 6,5 g IIa hinzu und rührt weitere 18 Std. bei 20°. Übliche Aufarbeitung (Kieselgel; Petrolether/Ethylacetat 1:1) liefert 1-(2'-Cyanbiphenylyl-4-methyl)-3-(2-phthalimidoethyl)-6-propyl-uracil, Öl, FAB 519.
b) Eine Lösung von 7,8 g 1-(2'-Cyanbiphenylyl-4-methyl)-3-(2-phthalimido-ethyl)-6-propyl-uracil und 3 ml Hydrazinhydrat in 60 ml Methanol wird 18 Std. bei 20° gerührt. Nach üblicher Aufarbeitung erhält man 1-(2'-Cyanbiphenylyl-4-methyl)-3-(2-aminoethyl)-6-propyl-uracil ("If"), Öl, FAB 389.
c) Eine Lösung von 2,4 g If und 0,76 ml Cyclohexylisocyanat in 20 ml Dioxan wird 3 Std. gekocht und dann eingedampft. Man erhält 1-(2'-Cyanbiphenylyl-4-methyl)-3-(2-N'-cyclohexylureido-ethyl)-6-propyl-uracil, FAB 514.
d) Eine Lösung von 2,4 g If und 0,6 ml Acetanhydrid in 15 ml Toluol wird 1 Std. bei 20° gerührt. Man dampft ein und erhält 1-(2'-Cyanbiphenyl-4-methyl)-3-(2-acetamidoethyl)-6-propyl-uracil, FAB 431.
e) Eine Lösung von 1 g If, 0,55 g Diphenylessigsäure, 0,5 g N-(3-Dimethylaminopropyl)-N'-ethylcarbodiimid-hydrochlorid und 0,53 g N-Methylmorpholin in 50 ml DMF wird 18 Std. bei 20° gerührt. Man gießt in Wasser und filtriert das erhaltene 1-(2'-Cyanbiphenylyl-4-methyl)-3-(2-diphenylacetamido-ethyl)-6-propyl-uracil ab; FAB 583.

### Beispiel 13

Analog Beispiel 10 erhält man aus den entsprechenden Nitrilen mit Trimethylzinnazid die nachstehenden 1-[2'-(1H-5-Tetrazolyl)-biphenylyl-4-methyl]-uracile:
3-Methyl-6-ethyl-, Trihydrat, F. 180°
3-o-Methoxycarbonyl-benzyl-6-butyl-, Nonahydrat, F. 135°
3-(2-Phthalimido-ethyl)-6-propyl, F. 135°
3-(2-Acetamido-ethyl)-6-propyl-, Tetrahydrat, F. 102°
3-(2-Diphenylacetamido-ethyl)-6-propyl-, Dihydrat, F. 212°
3-(2-N'-Cyclohexylureido-ethyl)-6-propyl-, F. 144°.

Die nachfolgenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

1. Di- bzw. Triazindione der Formel I 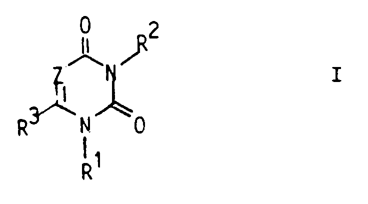 worin
Z CR⁴ oder N,
R¹ 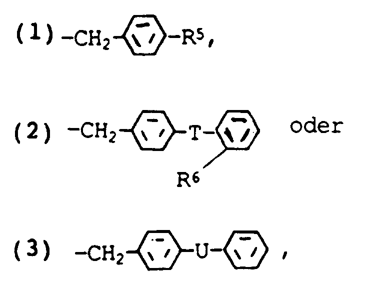
R² H, -C₁₋₁₀-Alkyl, -CₙH₂ₙ-COOR, -CₙH₂ₙ-CN, -CₙH₂ₙ-Ar, -CₙH₂ₙ-Het, -CₙH₂ₙ-CH=CH-Ar, -CₙH₂ₙ-CH=CH-Het, -CₙH₂ₙ-NR⁷R⁸, -C₂₋₆-Alkenyl, -CₙH₂ₙ-CO-N(R)₂, -CₙH₂ₙ-CO-R, -CₙH₂ₙ-Co-Ar, -CₘH₂ₘ-O-CON(R)₂ oder -CₘH₂ₘ-NR-CO-N(R)₂,
R³ H, A, Alkenyl mit 2-6 C-Atomen, eine Alkylgruppe mit 1-6 C-Atomen, worin eine CH₂-Gruppe durch ein O- oder S-Atom ersetzt ist, oder C₃₋₇-Cycloalkyl,
R⁴ sowie die Reste R unabhängig voneinander H, A oder C₃₋₇-Cycloalkyl,
R⁵ COOR, CN oder 1H-5-Tetrazolyl,
R⁶ COOR, CN, NO₂, NH₂, NHCOCF₃, NHSO₂CF₃ oder 1H-5-Tetrazolyl,
R⁷ H, -CO-R oder -CO-CH(Ar)₂,
R⁸ H,
R⁷ und R⁸ zusammen auch -CO-(o-C₆H₄)-CO-,
T fehlt, -NR-CO-, -CO-NR- oder -CH=CH-,
U -CH=C(COOR)-, -CH=C(CN)-, -CH=C(1H-5-Tetrazolyl)-, -O-CH(COOR)- oder -NR-CH(COOR)-,
m 1, 2, 3, 4, 5, 6, 7, 8, 9 oder 10,
n 0, 1, 2, 3, 4, 5, 6, 7, 8, 9 oder 10,
A C₁₋₆-Alkyl,
Ar eine unsubstituierte oder eine ein- oder zweifach durch A, Hal, CF₃,OH, OA, COOH, COOA, CN, NO₂, NH₂, NHA und/oder N(A)₂ substituierte Phenyl- oder Naphthylgruppe,
Het einen fünf- oder sechsgliedrigen heteroaromatischen Rest mit 1 bis 4 N-, O- und/oder S-Atomen, der auch mit einem Benzol- oder Pyridinring kondensiert sein kann und
Hal F, Cl, Br oder I bedeuten,
sowie ihre Salze.

2. a) 3-Methyl-6-propyl-1-[2'-(1H-5-tetrazolyl)-biphenylyl-4-methyl]-uracil;
b) 6-Butyl-3-methyl-1-[2'-(1H-5-tetrazolyl)-biphenylyl-4-methyl]-uracil.

3. Verfahren zur Herstellung von Di- bzw. Triazindionen der Formel I nach Anspruch 1 sowie ihrer Salze, dadurch gekennzeichnet, daß man
(a) eine Verbindung der Formel II
E-R¹ II
worin
E Cl, Br, I oder eine freie oder reaktionsfähig funktionell abgewandelte OH-Gruppe bedeutet und
R¹ die oben angegebene Bedeutung hat,
mit einer Verbindung der Formel III 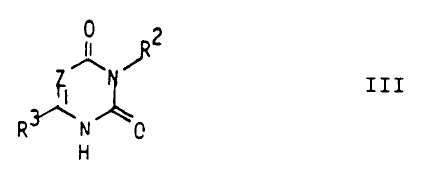 worin
Z, R², R³ und R⁴ die oben angegebene Bedeutung haben,
umsetzt,
oder
(b) zur Herstellung einer Verbindung der Formel I, worin T -NR-CO- oder -CO-NR- bedeutet, eine Verbindung der Formel IV 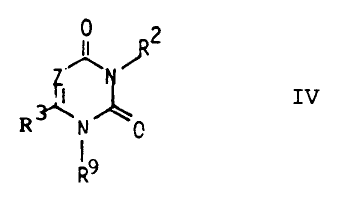 worin
R⁹ 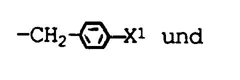
X¹ NH₂ oder COOH bedeuten, und
Z, R², R³ und R⁴ die oben angegebenen Bedeutungen haben,
oder ein reaktionsfähiges Derivat dieser Verbindung mit einer Verbindung der Formel V 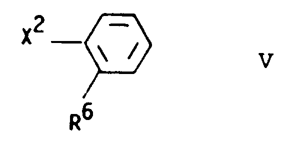 worin
X² COOH (falls X¹ NH₂ ist) oder NH₂ (falls X' COOH ist) bedeutet und
R⁶ die oben angegebene Bedeutung hat,
oder mit einem reaktionsfähigen Derivat dieser Verbindung umsetzt,
oder
(c) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹ und/oder R² in einen oder mehrere andere Reste R¹ und/oder R² umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verbindung der Formel I nach Anspruch 1 und ihre physiologisch unbedenklichen Salze zur Bekämpfung von Krankheiten.

7. Verwendung von Verbindungen der Formel I nach Anspruch 1 und/oder ihrer physiologisch unbedenklichen Salze zur Herstellung eines Arzneimittels.

8. Verwendung von Verbindungen der Formel I nach Anspruch 1 und/oder ihrer physiologisch unbedenklichen Salze bei der Bekämpfung von Krankheiten.
